# EUROPEAN PATENT SPECIFICATION

(11) **EP 1 731 132 B1**
(45) Date of publication and mention of the grant of the patent: **10.12.2008**
(21) Application number: 06118907.2
(22) Date of filing: 10.10.2001
(51) Int. Cl.: A61K 6/00, A61F 13/00, A61Q 11/00, A61K 8/11, A61K 8/21, A61K 8/24, A61K 8/73

(54) **Dental compositions for hypersensitive teeth**
Dentalzusammensetzungen für überempfindliche Zähne
Compositions dentaires pour dents hypersensibles

(30) Priority: 13.10.2000 US 240449 P
(43) Date of publication of application: 13.12.2006
(62) Divisional of application: 01979687.9
(73) Proprietor: BLOCK DRUG COMPANY, INC., West Trenton, New Jersey 08628 (US)
(72) Inventor: CURRO, Frederick, A, Emerson, NJ 07630 (US)
(74) Representative: Thompson, Clive Beresford

(56) References cited:
- US-A- 5 866 102
- US-A- 5 961 958

## Description

### FIELD OF INVENTION

The invention relates to compositions for the treatment of dentinal hypersensitivity and methods for the treatment of dentinal hypersensitivity using a substrate that is treated with a desensitizing agent.

### BACKGROUND OF THE INVENTION

Dentinal hypersensitivity is a temporary induced pain sensation produced when hypersensitive teeth are subjected to changes in temperature, pressure or chemical action. Hypersensitivity may occur whenever the dentin or cementum of a tooth is exposed by attrition or abrasion, or when the tooth's finer root surface is exposed by periodontal disease. Dentin generally contains channels, called tubules, that allow material and energy transport between the exterior of the dentin and the interior of the tooth where the nerve is located.

Many attempts have been made to control dentinal hypersensitivity. One approach is to reduce the excitability of the nerve in a sensitive tooth by altering the chemical environment of the nerve by using agents to make the nerve less sensitive. These agents are generally referred to as "nerve agents" or "nerve desensitizing agents." The most well known agent for this purpose is potassium nitrate, used in commercial dentifrices for sensitive teeth and discussed in U.S. Patent No. 3,863,006. U.S. Patent Nos. 4,631,185 and 4,751,072 disclose the desensitization of teeth using oral compositions comprising potassium salts such as potassium bicarbonate and potassium chloride, while U.S. Patent No. 4,990,327 describes the desensitization of teeth with strontium and fluoride ions. U.S. Patent No. 3,888,976 discloses the treatment of sensitive teeth using zinc and strontium ions.

Another approach to controlling dentinal hypersensitivity is to use agents that fully or partially occlude tubules. These agents are referred to as "tubule blocking agents." U.S. Patent No. 5,211,939 reports the use of charged polystyrene beads as tubule blocking agents. U.S. Patent Nos. 4,634,589 and 4,710,372 disclose the use of apatite as a tubule-blocking agent. U.S. Patent No. 5,589,159 teaches the use of Laponite or hectorite clay to seal dentinal tubules. U.S. Patent No. 5,270,031 discloses the use of a polyacrylic acid having a typical molecular weight from about 450,000 to about 4,000,000 as a tubule blocking agent. U.S. Patent No. 4,362,713 discloses the use of water-soluble or water-swellable polyelectrolytes, or salts thereof, as tubule blocking agents.

U.S. Patent No. 4,590,067 discloses the use of glucosamine, commonly known to have an anti-inflammatory effect when taken orally, in an oral composition for preventing and treating periodontal disease. U.S. Patent No. 4,855,128 discloses the use of chondroitin sulfate, commonly used in bone restoration applications, in a composition for inhibiting plaque. U.S. Patent No. 6,110,208 discloses a formulation for use as artificial skin containing a hyaluronic acid derivative. U.S. Patent No. 5,916,553 discloses the use of bone inducing protein complex for inducing the growth of bone in an animal.

It is known in the prior art to use microspheres onto which an active chemotherapeutic substance is adsorbed by chemical, electrostatic or ionic bonds to accelerate the rate at which wounds heal or bone regenerates, or for controlled sustained release of active chemotherapeutic substances in treatments. When the microspheres are hollow, they are both adsorbent and/or carriers of the functional groups, encapsulating the chemotherapeutic substance. When the microspheres are not hollow or contain pores on the surface, bonding with a pharmaceutical or cosmetic substance consists of adsorption into the pores or onto the surface. U.S. Patent No. 5,264,207 discloses microspheres of a polymer which act as carriers for one or more active pharmaceutical or cosmetic substances.

In the dentifrice art, U.S. Patent No. 5,565,206 discloses toothpaste compositions comprising particles having anti-microbial agents adsorbed onto the particles. U.S. Patent Nos. 5,211,939 and 5,250,288 describe the use of microspheres having charged polymeric particles adsorbed onto the surface to desensitize a hypersensitive tooth. Taking a different and opposite approach, U.S. Patent No. 4,157,387 discloses coating hard mineral substances, such as silica, with a water-soluble cationic polymer, forming a "coated abrasive," so that less of the therapeutic agent such as stannous fluoride, strontium chloride, and the like, is adsorbed by the coated abrasive and more of the therapeutic agent in free form is available for treatment of the teeth.

In the present invention, therapeutic agents are allowed to be directly adsorbed onto, or encapsulated within, a substrate, so that the substrate itself acts as a delivery vehicle of the therapeutic agent for the treatment of hypersensitive teeth.

### SUMMARY OF THE INVENTION

The invention provides a composition for desensitizing teeth comprising 1 to 70 wt.% of a substrate treated with at least a desensitizing agent, wherein the substrate treated with the desensitizing agent deposits or swells upon the dentinal surface and/or precipitates within the dentinal tubules, providing a concentrated and sustained release of the nerve desensitizing agent at the exposed dentinal surface and within the dentinal tubules.

The invention further provides a method for desensitizing hypersensitive teeth by applying thereto a desensitizing amount of an oral composition comprising a substrate treated with at least a desensitizing agent, wherein the substrate deposits or swells upon the dentinal surface and/or precipitates within the dentinal tubules, providing a concentrated amount of the desensitizing agent at the exposed dentinal surface and within the dentinal tubules.

This invention also discloses a method for preparing a composition for desensitizing teeth comprising a desensitizing amount of a composition comprising a substrate treated with at least a desensitizing agent.

### DETAILED DESCRIPTION OF THE INVENTION

By "substrate," as used herein, means microspheres of alumina, zeolite, titanium dioxide or phosphate glass on which the desensitizing agent is: (a) coated or adsorbed on the surface or attached to the surface by chemical, electrostatic or ionic bonds; and/or (b) encapsulated or impregnated within.

By "microspheres," as used herein, means hollow porous or non-porous particles, particulate, or materials that can be irregularly shaped or spherically shaped.

By "desensitizing agent," as used herein, means a material that reduces the excitability of the nerve in a sensitive tooth by: (a) altering the chemical environment of the nerve by using agents to make the nerve less sensitive; (b) promoting healing of the enamel or cemetum of the teeth; or (c) promoting regeneration of bone tissues to close the dentin tubules.

By "treated with," "treated," or "treating," as used herein interchangeably, means the process to cause or result in the encapsulation, impregnation, coating, or adsorption of the desensitizing agent within, or onto, the surface of the substrate.

The active component of the present invention is a substrate treated with a desensitizing agent or a mixture thereof.

The desensitizing agent for use in treating the substrate of the present invention includes a variety of anti-hypersensitivity agents or nerve desensitizing agents commonly known, i.e., agents that are neuroactive, and/or ions or salts which have a pain reducing or analgesic activity, are suitable for use to coat the substrate. Examples of nerve agents include without limitation, potassium or strontium salts, including potassium bicarbonate, potassium citrate, potassium chloride, potassium nitrate, strontium chloride, strontium acetate, strontium nitrate, and mixtures thereof.

In one embodiment, physiologically acceptable fluoride ions which have been reported to have a pain reducing or analgesic activity such as stannous fluoride, sodium fluoride, potassium fluoride, or mixtures thereof, are used as the desensitizing agents.

In yet another embodiment of the invention, materials commonly known for bone or tooth regeneration such as calcium phosphate-based compounds, are used as the desensitizing agents for treating the substrate. Calcium phosphate-based compounds, in particular synthetic hydroxyapatite represented by Ca₁₀(PO₄)₆(OH)₂, have the same composition as the inorganic main components of teeth and bones. In addition to the hydroxyapatite Ca₁₀(PO₄)₆(OH)₂ already mentioned, fluoroapatite Ca₁₀(PO₄)₆F₂, chloroapatite Ca₁₀(PO₄)₆Cl₂, tricalcium phosphate Ca₁₀(PO₄)₂ and various other kinds of known calcium phosphate-based compounds may be employed in the present invention. Such calcium phosphate-based compounds can be synthesized by known wet and dry methods.

In the same type of embodiment employing a bone or tooth regeneration material, biologically compatible calcium salts well-recognized in the art such as calcium gluconate, calcium carbonate, tricalcium and dicalcium phosphate, dolomite, and the like are used as the material to treat the substrate.

In yet a fourth embodiment, materials which are reported to have an effect on bone or cartilage renewal or rebuilding such as glucosamine and chondroitin sulfates are used as a desensitizing agent to treat the substrate. Glucosamine when used in the form of the salt with hydrochloric, sulfuric, phosphoric, or other biocompatible acid, is known to have an anti-inflammatory effect when taken orally or parenterally. Also useful are sugars and sugar derivatives of similar activity including 2-deoxy-D-glucose, 2-deoxy-D-galactose, mannose, D-mannosamine, D-galactosamine, glucosamine-6-phosphate, N-acetyl-D-glucosamine, N-acetyl-D-galactosamine, uridine diphosphate (UDP) glucose, UDP-N-acetylglucosamine, and the like.

The fifth embodiment employs another material also well known for its effects on healing bone fractures and cartilage defects in humans and other animals, i.e., bone morphogenetic protein (BMP) complexes. Bone protein complexes are typically isolated from almost any mammalian bone, and preferably calf bone due to its availability in large quantities.

In the sixth embodiment, hyaluronic acid derivatives and collagen, materials that are commonly for use in skin rebuilding, are used to treat the substrate. The hyaluronic acid and/or salts thereof and/or homologues, analogues, derivatives, complexes, esters, fragments, and sub-units of hyaluronic acid, are used in a form that is biocompatible. In one embodiment, the hyaluronic acid derivative is sodium hyaluronate.

"Beads" of alumina, zeolite, titanium dioxide, or phosphate glass can be used as the substrate for the desensitizing agent of the present invention. The substrate is generally of a size of about 10 microns or less, and preferably from about 0.5 to about 2 microns. The substrate particles can be spherical or irregular in shape. The particles can be hollow, porous, or non-porous.

There are various methods known in the prior art to coat or absorb the desensitizing agents onto the substrate or to impregnate/encapsulate the desensitizing agent within the substrate. For example, U.S. Patent No. 4,054,689 discloses a method to provide fluoride values in dentifrice formulation by treating with hydrogen fluoride vapors.

Another approach is to coat or absorb the desensitizing agents onto the surface of the substrate by using a technology commonly used in producing packing materials for liquid chromatography applications, in which the substrate is treated by methods such as sputtering, agglomeration by spray drying, or agglomeration by rolling and tumbling.

Yet another approach is to encapsulate the desensitizing agent or coating the substrate with the desensitizing agent by procedures generally described in Parrott, Pharmaceutical Technology, pp. 86-91 (Burgess Pub. Co. 1970); Deasy, Microencapsulation and Related Drug Procedures, pp. 1-60 (Marcel Dekker, Inc. 1984); Muller et al., J. Controlled Release, 20 (1992):237-246; Pekarek et al., Nature, vol. 367 (1994):258-60; Muller et al., Pharm. Pharmacol. Lett. vol. 3 (1993):67-70; and Juliano (ed.), Drug Delivery Systems (Oxford University Press 1980). These include solvent evaporation methods, with or without a surface active agent as necessary, coacervation in all its various forms, pan coating, air-suspension coating, press coating, spray-drying, rotational suspension-separation techniques, melt coating methods, interfacial polymerization, melt-granulation processes and any and all related methods that yield the desired substrate as described.

Depending on the method and the type of substrate used, the substrate may need to have charge opposite the desensitizing agent for coating, adsorption or encapsulation to occur and to provide a sufficient amount of desensitizing agent needed for the composition.

The substrate treated with a desensitizing agent, or mixtures thereof, is incorporated in the composition of the present invention in an amount from 1 to 70 wt.% depending on the type of substrate used. In one embodiment, the amount is 2 to 50 wt.%. In a second embodiment, it is 3 to 20 wt.%, and yet in a third embodiment, the substrate treated with a desensitizing agent is 5 to 15 wt.% of the formulation.

The desensitizing agent itself is incorporated in the final formulation in a desensitizing effective amount. This will vary depending on the particular type and form of oral composition, the substrate used, and other materials present. In one embodiment, the desensitizing agent is present in an amount of 0.1 to 15 wt.% of the final formulation. In another embodiment, it is present in an amount of 0.5 to 10 wt.% of the final formulation. In a third embodiment, it is present in an amount of 2 to 5 wt.% of the final formulation.

The compositions of the present invention are typically in the form of toothpastes or dentifrices to be brushed on the teeth. However, other delivery systems may also be used, including without limitation, tooth powder, mouthwash, lozenge, buccal adhesive patch, oral spray, coatings or chewing gum, and the like. For these delivery systems, one of skill in the art will be able to determine the amounts of the various agents described herein in order to achieve the desired effect.

Ingredients typically included in oral health care compositions may be used in the compositions in accordance with the invention. Optional ingredients include, without limitation, known desensitizing agents in free form such as potassium nitrate, potassium chloride, potassium bicarbonate and strontium chloride. Other ingredients include without limitation, abrasive polishing materials, sudsing agents, flavoring agents, humectants, binders, sweetening agents, and water.

Abrasives which may optionally be used in the compositions of the invention include without limitation, alumina and hydrates thereof, such as alpha alumina trihydrate, magnesium trisilicate, magnesium carbonate, aluminosilicate, such as calcined aluminum silicate and aluminum silicate, calcium carbonate, zirconium silicate, polymethylmethacrylate, powdered polyethylene, silica xerogels, hydrogels and aerogels and the like. Also suitable as abrasive agents are calcium pyrophosphate, insoluble sodium metaphosphate, calcium carbonate, dicalcium orthophosphate, particular hydroxyapatite, and the like. Depending on the form that the oral composition is to take and whether the substrate is an abrasive-based material, the abrasive may be present in an amount of from 0 to 70 wt.%.

Humectants contemplated for use include without limitation, glycerol, polyol, sorbitol, polyethylene glycols, propylene glycol, hydrogenated partially hydrolyzed polysaccharides, and the like. The humectants are generally present in amounts of from 0 to 80 wt.%, and preferably 5 to 70 wt.% for toothpastes. Thickeners suitable for use in the invention include without limitation, silica. Thickeners may be present at a level from 0.1 to 20 wt.%.

Binders suitable for use in the compositions of the invention include without limitation, hydroxyethyl cellulose, and hydroxypropyl cellulose, as well as xanthan gums, Iris moss and gum tragacanth. Binders may be present in the amount from 0.01 to 5 wt.%.

Sweeteners suitable for use may be present at levels of about 0.1 to 10 wt.%, and include without limitation, saccharin and xylitol.

Fluoride sources commonly used in oral health care compositions such as sodium fluoride, stannous fluoride, sodium monofluorophosphate, zinc ammonium fluoride, tin ammonium fluoride, calcium fluoride and cobalt ammonium fluoride may be included for providing anti-caries benefit, Preferred compositions of the invention include a fluoride source. Fluoride ions are typically provided at a level of from 0 to 1500 ppm, preferably 50 to 1500 ppm, although higher levels up to about 3000 ppm may be used.

Surfactants, such as a soap, anionic, nonionic, cationic, amphoteric and/or zwitterionic, may be present within the range of 0 to 15 wt.%, preferably 0.1 to 15 wt.%, more preferably 0.25 to 10 wt.%. Anionic and/or nonionic surfactants are most preferred, such as sodium lauroyl sulfate, sodium lauroyl sarcosinate and sodium dodecylbenzene sulfonate. Flavors are usually included in low amounts, such as from about 0.01 to 5 wt.%, especially from 0.1 to 5 wt.%.

Antibacterial agents include without limitation, phenolics and salicylamides, and sources of certain metal ions such as zinc, copper, silver and stannous (e.g. zinc, copper and stannous chloride, and silver nitrate) may also be, and preferably are, included.

Dyes/colorants suitable for oral health care compositions, e.g., FD&C Blue #1, FD&C Yellow #10, FD&C Red #40, etc., may be included in the compositions of this invention.

Various other optional ingredients may be included in the compositions of the invention such as preservatives, vitamins such as vitamin C and E, other anti-plaque agents such as stannous salts, copper salts, strontium salts and magnesium salts. Also included may be pH adjusting agents; anti-caries agents such as calcium glycerophosphate, sodium trimetaphosphate; anti-staining compounds such as silicone polymers, plant extracts, and mixtures thereof. Additionally, polymers, particularly anionic polymers, such as polycarboxylates or polysulfonates, or polymers containing both a carboxylate and a sulfonate moiety, phosphonate polymers or polyphosphates, may be included.

Ingredients mentioned above are conventional ingredients suitable for oral care compositions e.g., toothpastes, gels, gums, powders, etc. Except where otherwise noted, references to toothpastes are to be construed as applying to gels as well.

The compositions of this invention are prepared by conventional methods of making oral health care formulations by mixing the ingredients in an order that is convenient to achieve the desired effects. For instance, forming a gel with gelling agent and water and then adding other ingredients in toothpaste and gel dentifrice embodiments. In dentifrice form, the composition may be packaged in a conventional plastic laminate or metal tube or a dispenser, or present in separate phases to enhance appearance. It may be applied to dental surfaces by any physical means, such as a toothbrush, fingertip or by an applicator directly to the sensitive area. Solid dosage form examples include pastilles, lozenges, chewing gums, tablets, mouthstrips, balms and the like.

## Claims

1. A composition for reducing dentinal hypersensitivity comprising from 1 to 70 wt.% of an alumina, zeolite, titanium dioxide or phosphate glass substrate treated with a desensitizing agent, wherein said desensitizing agent is encapsulated or impregnated within, or coated and adsorbed onto the surface of, the substrate, and is present in an amount of 0.1 to 15 wt.% of the final formulation.

2. The composition according to claim 1, wherein said substrate is a porous material.

3. The composition according to claim 1, wherein said substrate is a non-porous material.

4. The composition according to any of claims 1 to 3, wherein said substrate is 10 µm (microns) or less in size.

5. The composition according to any of claims 1 to 4, wherein said desensitizing agent is selected from:
(a) a potassium salt, a strontium salt, or mixtures thereof;
(b) fluoride ions from stannous fluoride, sodium fluoride, potassium fluoride, or mixtures thereof;
(c) a calcium phosphate-based compound;
(d) a calcium salt; and
(e) a material selected from glucosamine, glucosamine derivative, chondroitin sulfate, collagen, hyaluronic acid and derivatives, bone morphogenetic protein complexes, and mixtures thereof.

6. The composition according to claim 5, wherein said calcium phosphate-based compound is selected from hydroxyapatite Ca₁₀(PO₄)₆(OH)₂, fluoroapatite Ca₁₀(PO₄)₆F₂,chloroapatite Ca₁₀(PO₄)₆Cl₂, tricalcium phosphate Ca₁₀(PO₄)₂, and mixtures thereof.

7. The composition according to claim 5, wherein said calcium salt is selected from calcium gluconate, calcium carbonate, tricalcium phosphate, dicalcium phosphate, dolomite, and mixtures thereof.

8. The composition according to claim 5, wherein said glucosamine derivative is selected from 2-deoxy-D-glucose, 2-deoxy-D-galactose, mannose, D-mannosamine, D-galactosamine, glucosamine-6-phosphate, N-acetyl-D-glucosamine, N-acetyl-D-galactosamine, uridine diphosphate (UDP) glucose, UDP-N-acetylglucosamine, and mixtures thereof.

9. The composition according to claim 5, wherein the desensitizing agent is selected from potassium bicarbonate, potassium citrate, potassium chloride, potassium nitrate, strontium chloride, strontium acetate, strontium nitrate, and mixtures thereof.

10. The composition according to any one of claims 1 to 9, wherein said substrate treated with a desensitizing agent is present in an amount of from 2 to 50 wt.%, and said desensitizing agent is in an amount of 0.5 to 10 wt.%, of the final formulation.

11. The composition according to any of claims 1 to 10 in the form of a dentifrice.

12. Use of a composition according to any of claims 1 to 11 in the manufacture of a medicament for reducing dentinal hypersensitivity in a sensitive tooth.

## Patentansprüche

1. Zusammensetzung zum Reduzieren der Dentin-Empfindlichkeit, umfassend von 1 bis 70 Gew.% eines Aluminiumoxid-, Zeolith-, Titandioxid- oder Phosphatglassubstrats, das mit einem Desensibilisierungsmittel behandelt ist, wobei dieses Desensibilisierungsmittel mit dem Substrat verkapselt oder imprägniert ist, oder auf die Oberfläche davon beschichtet oder absorbiert ist, und in einer Menge von 0,1 bis 15 Gew.% der Endformulierung vorhanden ist.

2. Zusammensetzung gemäß Anspruch 1, wobei das Substrat ein poröses Material ist.

3. Zusammensetzung gemäß Anspruch 1, wobei das Substrat ein nicht-poröses Material ist.

4. Zusammensetzung gemäß irgendeinem der Ansprüche 1 bis 3, wobei das Substrat 10 µm (Mikrometer) oder weniger groß ist.

5. Zusammensetzung gemäß irgendeinem der Ansprüche 1 bis 4, wobei dieses Desensibilisierungsmittel ausgewählt ist aus:
(a) einem Kaliumsalz, einem Strontiumsalz oder Gemischen daraus;
(b) Fluorionen aus Stannofluorid, Natriumfluorid, Kaliumfluorid oder Gemischen daraus;
(c) einer auf Calciumphosphat basierenden Verbindung;
(d) einem Kalziumsalz; und
(e) einem Material, ausgewählt aus Glucosamin, Glucosaminderivat, Chondroitinsulfat, Kollagen, Hyaluronsäure und Derivaten, Knochenmorphogeneseproteinkomplexen und Gemischen daraus.

6. Zusammensetzung gemäß Anspruch 5, wobei diese auf Calciumphosphat basierende Verbindung ausgewählt ist aus Hydroxyapatit Ca₁₀(PO₄)₆(OH)₂, Fluorapatit Ca₁₀(PO₄)₆F₂, Chlorapatit Ca₁₀(PO₄)₆Cl₂, Tricalciumphosphat Ca₁₀(PO₄)₂ und Gemischen davon.

7. Zusammensetzung gemäß Anspruch 5, wobei dieses Calciumsalz ausgewählt ist aus Calciumgluconat, Calciumcarbonat, Tricalciumphosphat, Dicalciumphosphat, Dolomit und Gemischen daraus.

8. Zusammensetzung gemäß Anspruch 5, wobei dieses Glucosaminderivat ausgewählt ist aus 2-Desoxy-D-glucose, 2-Desoxy-D-galactose, Mannose, D-Mannosamin, D-Galactosamin, Glucosamin-6-phosphat, N-Acetyl-D-glucosamin, N-Acetyl-D-galactosamin, Uridindiphosphat (UDP)-glucose, UDP-N-Acetylglucosamin und Gemischen daraus.

9. Zusammensetzung gemäß Anspruch 5, wobei das Desensibilisierungsmittel ausgewählt ist aus Kaliumbicarbonat, Kaliumcitrat, Kaliumchlorid, Kaliumnitrat, Strontiumchlorid, Strontiumacetat, Strontiumnitrat und Gemischen daraus.

10. Zusammensetzung gemäß irgendeinem der Ansprüche 1 bis 9, wobei dieses mit einem Desensibilisierungsmittel behandelte Substrat in einer Menge von ungefähr 2 bis 50 Gew.% vorhanden ist und dieses Desensibilisierungsmittel in einer Menge von ungefähr 0,5 bis 10 Gew.% der Endformulierung vorhanden ist.

11. Zusammensetzung gemäß irgendeinem der Ansprüche 1 bis 10 in Form einer Zahnpasta.

12. Verwendung einer Zusammensetzung gemäß irgendeinem der Ansprüche 1 bis 11 zur Herstellung eines Medikaments zum Reduzieren der Dentin-Empfindlichkeit eines empfindlichen Zahns.

## Revendications

1. Composition pour réduire l'hypersensibilité dentinaire comprenant 1 à 70 % en poids d'un substrat à base d'alumine, de zéolithe, de dioxyde de titane ou de verre de phosphate, traité avec un agent de désensibilisation, dans laquelle ledit agent de désensibilisation est encapsulé ou imprégné dans le substrat ou enrobé et adsorbé sur la surface du substrat, et est présent en une quantité de 0,1 à 15 % en poids de la formule finale.

2. Composition selon la revendication 1, dans laquelle ledit substrat est un matériau poreux.

3. Composition selon la revendication 1, dans laquelle ledit substrat est un matériau non poreux.

4. Composition selon l'une quelconque des revendications 1 à 3, dans laquelle la taille dudit substrat est de 10 µm (microns) ou moins.

5. Composition selon l'une quelconque des revendications 1 à 4, dans laquelle ledit agent de désensibilisation est choisi parmi :
(a) un sel de potassium, un sel de strontium ou leurs mélanges ;
(b) des ions fluorures provenant du fluorure stanneux, du fluorure de sodium, du fluorure de potassium ou de leurs mélanges ;
(c) un composé à base de phosphate de calcium ;
(d) un sel de calcium ; et
(e) une substance choisie parmi la glucosamine, un dérivé de glucosamine, le sulfate de chondroïtine, le collagène, l'acide hyaluronique et ses dérivés, des complexes de protéines osseuses morphogénétiques et leurs mélanges.

6. Composition selon la revendication 5, dans laquelle ledit composé à base de phosphate de calcium est choisi parmi l'hydroxyapatite Ca₁₀(PO₄)₆(OH)₂, la fluoroapatite Ca₁₀(PO₄)₆F₂, la chloroapatite Ca₁₀(PO₄)₆Cl₂, le phosphate tricalcique Ca₁₀(PO₄)₂ et leurs mélanges.

7. Composition selon la revendication 5, dans laquelle ledit sel de calcium est choisi parmi le gluconate de calcium, le carbonate de calcium, le phosphate tricalcique, le phosphate dicalcique et leurs mélanges.

8. Composition selon la revendication 5, dans laquelle ledit dérivé de glucosamine est choisi parmi le 2-désoxy-D-glucose, le 2-désoxy-D-galactose, le mannose, la D-mannosamine, la D-galactosamine, la glucosamine-6-phosphate, la N-acétyl-D-glucosamine, la N-acétyl-D-galactosamine, l'uridine diphosphate (UDP) glucose, l'UDP-N-acétylglucosamine et leurs mélanges.

9. Composition selon la revendication 5, dans laquelle l'agent de désensibilisation est choisi parmi le bicarbonate de potassium, le citrate de potassium, le chlorure de potassium, le nitrate de potassium, le chlorure de strontium, l'acétate de strontium, le nitrate de strontium et leurs mélanges.

10. Composition selon l'une quelconque des revendications 1 à 9, dans laquelle ledit substrat traité avec un agent de désensibilisation est présent en une quantité de 2 à 50 % en poids et ledit agent de désensibilisation est présent en une quantité de 0,5 à 10 % en poids de la formule finale.

11. Composition selon l'une quelconque des revendications 1 à 10, sous la forme d'un dentifrice.

12. Utilisation d'une composition selon l'une quelconque des revendications 1 à 11, dans la fabrication d'un médicament destiné à réduire l'hypersensibilité dentinaire d'une dent sensible.
